# EUROPÄISCHE PATENTSCHRIFT

(11) **EP 4 447 813 B1**
(45) Veröffentlichungstag und Bekanntmachung des Hinweises auf die Patenterteilung: **20.08.2025**
(21) Anmeldenummer: 22840584.1
(22) Anmeldetag: 16.12.2022
(51) Int. Cl.: A61B 8/00, G01F 1/66, G01F 1/667, G01N 29/22

(54) **VORRICHTUNG FÜR EIN MEDIZINISCHES BEHANDLUNGSGERÄT ZUR MESSUNG DES FLUSSES VON FLÜSSIGKEITEN IN EINEM EINZULEGENDEN LUMEN UND EIN ENSEMBLE MIT EINER ENTSPRECHENDEN VORRICHTUNG UND EINEM LUMEN**
DEVICE FOR A MEDICAL TREATMENT APPARATUS FOR MEASURING THE FLOW OF FLUIDS IN A LUMEN TO BE INSERTED, AND ENSEMBLE HAVING A CORRESPONDING DEVICE AND A LUMEN
DISPOSITIF POUR UN APPAREIL DE TRAITEMENT MÉDICAL DESTINÉ À MESURER L'ÉCOULEMENT DE FLUIDES DANS UNE LUMIÈRE À INSÉRER ET ENSEMBLE COMPORTANT UN DISPOSITIF CORRESPONDANT ET UNE LUMIÈRE

(30) Priorität: 16.12.2021 DE 102021214511
(43) Veröffentlichungstag der Anmeldung: 23.10.2024
(73) Patentinhaber: Fresenius Medical Care Deutschland GmbH, 61352 Bad Homburg (DE)
(72) Erfinder: STÖCKERL, Tobias, 61352 Bad Homburg (DE); URBAN, Martin, 61352 Bad Homburg (DE); PETERS, Arne, 61352 Bad Homburg (DE)
(74) Vertreter: RCD-Patent Giesen, Schmelcher & Griebel Patentwanwälte PartG mbB
(86) Internationale Anmeldenummer: PCT/EP2022/086293
(87) Internationale Veröffentlichungsnummer: WO 2023/111237

(56) Entgegenhaltungen:
- WO-A1-2008/053193
- DE-A1- 102011 084 171
- US-A- 4 545 259
- US-A- 5 463 906
- US-A1- 2007 062 305
- US-A1- 2013 104 667
- US-A1- 2019 285 450
- US-A1- 2021 325 218

## Beschreibung

Die Erfindung betrifft eine Vorrichtung für ein medizinisches Behandlungsgerät zur Messung des Flusses von Flüssigkeiten in einem einzulegenden Lumen und ein Ensemble mit einer entsprechenden Vorrichtung und einem Lumen.

### Hintergrund

In medizinischen Behandlungsgeräten ist es häufig erforderlich den Fluss eines Mediums zu messen. Dabei tritt das Problem auf, dass alle Elemente eines medizinischen Behandlungsgerätes, die mit einem zum behandelnden Patienten in Berührung kommen könnten, d.h. insbesondere flüssigkeitsführende Lumen, sterilisierbar sein müssen.

Aus dem Stand der Technik sind verschiedene Einrichtungen zur Messung eines Flusses bekannt.

So ist es möglich den Fluss in einer festen Anordnung mittels Ultraschalles zu messen. Allerdings sind solche Anordnungen fest installiert, sodass z.B. bei einem medizinischen Behandlungsgerät die feste Anordnung in einen Fluidkreislauf integriert werden muss. Dies führt dazu, dass die Anordnung für die Verwendung an einem Patienten sterilisiert werden muss. Dies ist zeitaufwändig aber auch fehlerbehaftet. Zudem ergibt sich bei diesen festen Anordnungen häufig der Nachteil, dass dort ein seitliches Einströmen /Ausströmen in einem 90° Winkel zur Messstrecke vorgesehen ist. Solche Winkel führen aber häufig zu starken Verwirbelungen, die die Messung beeinträchtigen können.

Um diesem Problem zu entgehen, wurden in der Vergangenheit Lösungen entwickelt, bei denen mittels einer klammerartigen Ausgestaltung ein Einweglumen beobachtet wurde. Beispielhaft sei auf die US Patentanmeldung US 2013 / 104 667 A1 verweisen, aus der eine nachträglich um ein Lumen fixierbare ultraschall-basierte Flussratenmesseinrichtung bekannt ist. Diese Einrichtung muss jedoch präzise auf den Lumendurchmesser abgestimmt sein.

Dabei wird jedoch prinzipbedingt senkrecht zum Fluss (also zur Flussrichtung) ein Signal in das Lumen eingekoppelt oder gemessen.

Typisch dabei sind Anordnungen, bei denen zwei Elemente der Messanordnung (z.B. Ultraschallwandler) entlang des Lumens versetzt sind, d.h. ein Element liegt stromaufwärts des anderen Elements. Die so gebildete Messstrecke ist typischerweise nur unwesentlich länger, da der Versatz üblicherweise so gering ist, dass maximal ein Winkel von 45° gebildet wird zwischen der Flussrichtung und der Verbindungslinie zwischen den Elementen der Messanordnung, wodurch sich die Messstrecke im Vergleich zu senkrecht auf maximal ca. das 1,4-fache des Durchmessers des Lumens verlängert.

Es zeigt sich, dass solche Messungen zwar möglich sind, jedoch auf Grund der Messanordnung prinzipiell eine geringe Auflösung besitzen. Dies liegt darin begründet, dass die zu bestimmenden Zeitintervalle deutlich kleiner sind als bei einer Messung in / gegen Flussrichtung. Darüber hinaus ist die Sensitivität der Messung auch bereits deswegen gering, weil das zeitgleich in der Messstrecke gegenwärtige Flüssigkeitsvolumen durch die beinahe senkrechte Anordnung beinahe minimal ist, im Vergleich zu Anordnungen mit anderen Winkeln bzw. deren Messstrecken. Zudem zeigt sich, dass die Ankopplung problematisch ist, da zum einen die Ultraschallwandler eine gute Ankopplung besitzen müssen, anderseits aber der Anpressdruck der klammerartigen Ausgestaltung nicht zu hoch sein darf, um einerseits in der klinischen Praxis durch einen menschlichen Nutzer überhaupt handhabbar zu sein und anderseits nicht die Messung zu gefährden. Soll eine solche Anordnung für unterschiedliche Lumendurchmesser praktikabel sein, ist dies nur sehr eingeschränkt möglich. Zudem ist bei den bisherigen Ausgestaltungen das Handling eines Einweglumens mit mehreren Arbeitsschritten verbunden, um wegen der geringen Auflösung die definierten Anpresseigenschaften von Ultraschallwandlern an das Einweglumen bereitzustellen, bevor eine Messung möglich ist.

### Aufgabe

Ausgehend hiervon ist es eine Aufgabe der Erfindung eine verbesserte Anordnung bereitzustellen, die bei einfacher Handhabung eine hohe Messauflösung für verschiedene Lumendurchmesser bietet. Eine alternative oder zusätzliche Aufgabe der Erfindung ist es, "scharfkantige" oder unstete Geometrien im Flüssigkeitslumen stromaufwärts der Messstrecke zu vermeiden, welche so nahe an der Messstrecke liegen, dass Verwirbelungen im Bereich der Messstrecke auftreten.

### Kurzdarstellung der Erfindung

Die Aufgabe wird gelöst durch eine Vorrichtung für ein medizinisches Behandlungsgerät zur Messung des Flusses von Flüssigkeiten in einem einzulegenden Lumen, aufweisend eine Aufnahme und einen ersten Ultraschallwandler, wobei die Aufnahme in der Vorrichtung eine erste Führungsseite und eine zweite Führungsseite aufweist, sodass ein eingelegtes Lumen in seitlicher Projektion in etwa einen trapezförmigen Verlauf aufweist, wobei der erste Ultraschallwandler an einer ersten Trapezecke angeordnet ist, und wobei der erste Ultraschallwandler im Betrieb dazu ausgestaltet ist, eine Messung in bzw. gegen Flussrichtung im Lumen durchzuführen.

Diese Ausgestaltung stellt bei einfacher Handhabung eine hohe Messauflösung für verschiedene Lumendurchmesser bereit.

In einer Ausführungsform der Erfindung weist die Vorrichtung weiterhin einen zweiten Ultraschallwandler auf, wobei der zweite Ultraschallwandler an einer zweiten Trapezecke angeordnet ist, und wobei der zweite Ultraschallwandler im Betrieb dazu ausgestaltet ist, eine Messung in bzw. gegen Flussrichtung im Lumen in Gegenrichtung zum ersten Ultraschallwandler durchzuführen.

In einer Variante dieser Ausführungsform ist die Vorrichtung dazu ausgestaltet, dass der zweite Ultraschallwandler in Zusammenspiel mit dem ersten Ultraschallwandler Messungen durchführt, sodass Messungen in beide Richtungen ausgeführt werden.

Diese Ausgestaltung bietet den Vorteil, dass sowohl in Fluss- als auch Gegenflussrichtung gemessen werden kann, sodass eine höhere Messgenauigkeit zur Verfügung gestellt werden kann.

Gemäß einer Ausführungsform der Erfindung wird der Fluss mittels einer Laufzeitmethode gemessen, bei der die Laufzeit eines Ultraschallsignals von einem Ultraschallwandler entlang der Messstrecke einmal mit der Flussrichtung und einmal gegen die Flussrichtung gemessen wird. Dabei kann eine Reflexion am Ende der Messstrecke zurück in Ausgangsrichtung das Messprinzip bei einer Anordnung mit nur einem Ultraschallwandler, welcher erst als Sender und dann als Empfänger auftritt, ermöglichen und bei einer Anordnung mit zwei Ultraschallwandlern können beide wechselseitig als Sender bzw. Empfänger agieren.

Gemäß einer Ausführungsform der Erfindung ist die zu vermessende Flüssigkeit Blut.

An die Messung von Eigenschaften von Blut werden besondere Anforderungen gestellt. Damit kann die Vorrichtung insbesondere für Blutbehandlungsgeräte, z.B. Dialyse- und Apheresegeräte, und im Kontext von extrakorporalen Blutkreislaufen, z.B. für Vorrichtungen zur Herz- und/oder Lungenunterstützung oder Ersatztherapie, verwendet werden.

Gemäß einer Ausführungsform der Erfindung ist die zu vermessende Flüssigkeit Blut, eine andere biologische Flüssigkeit oder Dialyse-Substituat oder eine medizinische Flüssigkeit wie ein Flüssigmedikament oder ein Gemisch der vorgenannten Flüssigkeiten.

Damit kann die Vorrichtung insbesondere für Blutbehandlungsgeräte, z.B. Dialyse- und Apheresegeräte, und im Kontext von extrakorporalen Blutkreislaufen, z.B. für Vorrichtungen zur Herz- und/oder Lungenunterstützung oder Ersatztherapie, verwendet werden.

Gemäß einer Ausführungsform der Erfindung ist die zu vermessende Flüssigkeit Dialysat oder eine andere physiologische Ersatzflüssigkeit oder eine medizinische Flüssigkeit wie ein Flüssigmedikament oder ein Gemisch der vorgenannten Flüssigkeiten.

Damit kann die Vorrichtung insbesondere für Blutbehandlungsgeräte wie Dialysegeräte, Dialysespendeanordnungen und Apheresegeräte, verwendet werden.

In einer weiteren Ausführungsform der Erfindung ist die zu vermessende Flüssigkeit Flüssignahrung z.B. zur Sondenernährung.

In einer weiteren Ausführungsform der Erfindung weist die Vorrichtung weiterhin eine Klemme auf, die ein eingelegtes Lumen selektiv durch Abklemmen unterbrechen kann.

Damit wird ermöglicht, neben Messfunktionen auch Schaltfunktionen, z.B. in einem erkannten Fehlerfall, bereitzustellen.

Gemäß einer weiteren Ausführungsform der Erfindung ist das Trapez gleichschenklig.

Mit einer gleichschenkligen Anordnung kann die Messtechnik spiegelbildlich und damit mit gleichartigen Teilen aufgebaut werden, wodurch Entwicklungs- als auch Produktionskosten sinken.

In noch einer weiteren Ausführungsform der Erfindung weist die Vorrichtung weiterhin eine externe Auswerteeinrichtung zur Steuerung und Auswertung von Ultraschallwandlern auf.

D.h. mittels der Auswerteeinrichtung kann nun eine integrierte Baueinheit zur Verfügung gestellt werden.

Gemäß noch einer weiteren Ausführungsform der Erfindung weist die Vorrichtung weiterhin mindestens einen weiteren Sensor zur Messung eines weiteren Parameters der Flüssigkeit auf.

Durch die Integration weiterer Sensoren lassen sich kostengünstige Baugruppen mit weiteren Mess-Eigenschaften aufbauen.

In einer weiteren Ausführungsform der Erfindung ist die Vorrichtung weiterhin dazu eingerichtet, Mikroblasen in der Flüssigkeit, welche sich in einem eingelegten Lumen befindet, zu erkennen, z.B. an Hand von Fluktuation in gemessenen Flüssen.

Mikroblasen können Zeichen beginnender Probleme als auch Fehler sein, sodass das Erkennen von Mikroblasen die Sicherheit erhöht. Hierbei kommt vorteilhaft zum Tragen, dass die Anordnung eine Messung an einem größeren Flüssigkeitsvolumen vorsieht als in den beinahe senkrechten (oder auch unter einem Winkel von 45°) bekannten Messanordnungen. Somit kann auch ein größeres Volumen zeitgleich auf die Gegenwart von Mikroblasen hin gemessen werden. In einer Variante der Ausführungsform wird die Gegenwart von Mikroblasen anhand von Fluktuationen im Messignal, aus welchem der Fluss ermittelt wird (z.B. Laufzeitdifferenz von Schallsignalen mit und entgegen der Flussrichtung), aber nicht zwingend direkt aus der somit gewonnen Messgröße allein ermittelt, sondern es wird eine zusätzliche Größe zur Auswertung der Fluktuationen im Messsignal herangezogen. Diese zusätzliche Messgröße kann z.B. eine Amplitudeninformation des übertragenen Ultraschallsignals sein.

In einer weiteren Ausführungsform ist die Vorrichtung dazu eingerichtet, zusätzlich eine Messgröße zu ermitteln, welche mit der Dichte der zu messenden Flüssigkeit korreliert.

In einer weiteren Ausführungsform ist die Vorrichtung dazu eingerichtet, zusätzlich eine Messgröße zu ermitteln, welche mit der Temperatur der zu messenden Flüssigkeit korreliert.

Gemäß noch einer weiteren Ausführungsform der Erfindung sind die erste Führungsseite und die zweite Führungsseite relativ zueinander verschiebbar.

Hierdurch kann die Bandbreite von Lumendurchmesser besonders gut vergrößert werden.

In noch einer weiteren Ausführungsform der Erfindung übt die erste Führungsseite und die zweite Führungsseite auf ein eingelegtes Lumen eine definierte und / oder einstellbare oder Kraft aus.

Hierdurch kann der Ultraschallwandler in einem günstigen Arbeitsbereich Messungen mit hoher Genauigkeit zur Verfügung stellen.

Gemäß noch einer weiteren Ausführungsform der Erfindung ist die eine Aufnahme für verschiedene Durchmesser von einlegbaren Lumen ausgelegt.

In noch einer weiteren Ausführungsform der Erfindung kann die erste Führungsseite und die zweite Führungsseite schubladenartig gemeinsam relativ zu einem Gehäuse verschoben werden.

Hierdurch kann eine besonders gute Handhabbarkeit erreicht werden.

Gemäß einer weiteren Ausgestaltung der Erfindung wird auch ein Ensemble mit einer erfindungsgemäßen Vorrichtung zur Messung und einem Lumen zur Verfügung gestellt.

Diese Ausgestaltung stellt bei einfacher Handhabung eine hohe Messauflösung für verschiedene Lumendurchmesser bereit.

Gemäß einer Ausführungsform der Erfindung ist das Lumen ein Einwegschlauch.

In einer Ausführungsform der Erfindung ist das Lumen elastisch.

Damit lassen sich herkömmliche Schlauchsysteme als auch Einwegartikel auch mit der erfindungsgemäßen Vorrichtung kombinieren.

Weitere vorteilhafte Ausgestaltungen sind Gegenstand der jeweils abhängigen Ansprüche, der Figuren und der Beschreibung.

### Kurzdarstellung der Figuren

Im Folgenden wird die Erfindung anhand einer Zeichnung und Ausführungsbeispielen näher erläutert. Die Zeichnung ist eine schematische Darstellung und nicht maßstabsgetreu. Die Zeichnung schränkt die Erfindung in keiner Weise ein.

Es zeigen:
- Fig. 1: eine schematische Darstellung von Ausführungsformen der Erfindung ohne eingelegtes Lumen,
- Fig. 2: eine schematische Darstellung von Ausführungsformen der Erfindung mit eingelegtem Lumen, und
- Fig. 3a und 3b: eine schematische Darstellung von weiteren Aspekten von Ausführungsformen der Erfindung.

### Ausführliche Darstellung der Erfindung

Nachfolgend wird die Erfindung eingehender unter Bezugnahme auf die Figuren dargestellt werden. Dabei ist anzumerken, dass unterschiedliche Aspekte beschrieben werden, die jeweils einzeln oder in Kombination zum Einsatz kommen können. D.h. jeglicher Aspekt kann mit unterschiedlichen Ausführungsformen der Erfindung verwendet werden, soweit nicht explizit als reine Alternative dargestellt.

Weiterhin wird nachfolgend der Einfachheit halber in aller Regel immer nur auf eine Entität Bezug genommen. Soweit nicht explizit vermerkt, kann die Erfindung aber auch jeweils mehrere der betroffenen Entitäten aufweisen. Insofern ist die Verwendung der Wörter "ein", "eine" und "eines" nur als Hinweis darauf zu verstehen, dass in einer einfachen Ausführungsform zumindest eine Entität verwendet wird.

Soweit nachfolgend Verfahren beschrieben werden, sind die einzelnen Schritte eines Verfahrens in beliebiger Reihenfolge anordbar und/oder kombinierbar, soweit sich durch den Zusammenhang nicht explizit etwas Abweichendes ergibt. Weiterhin sind die Verfahren - soweit nicht ausdrücklich anderweitig gekennzeichnet - untereinander kombinierbar.

Angaben mit Zahlenwerten sind in aller Regel nicht als exakte Werte zu verstehen, sondern beinhalten auch eine Toleranz von +/- 1 % bis zu +/- 10 %.

Soweit in dieser Anmeldung Normen, Spezifikationen oder dergleichen benannt werden, werden zumindest immer die am Anmeldetag anwendbaren Normen, Spezifikationen oder dergleichen in Bezug genommen. D.h. wird eine Norm / Spezifikation etc. aktualisiert oder durch einen Nachfolger ersetzt, so ist die Erfindung auch hierauf anwendbar.

In den Figuren sind verschiedene Ausführungsformen dargestellt.

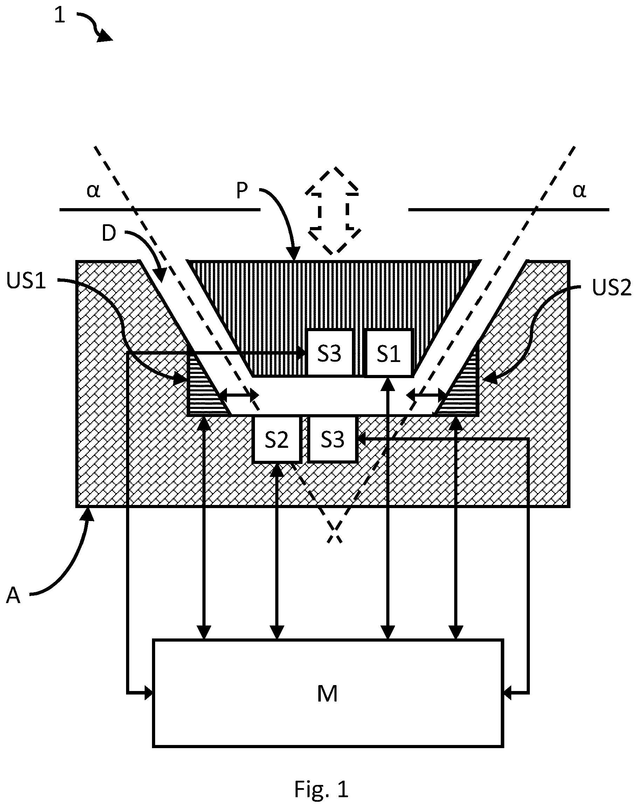

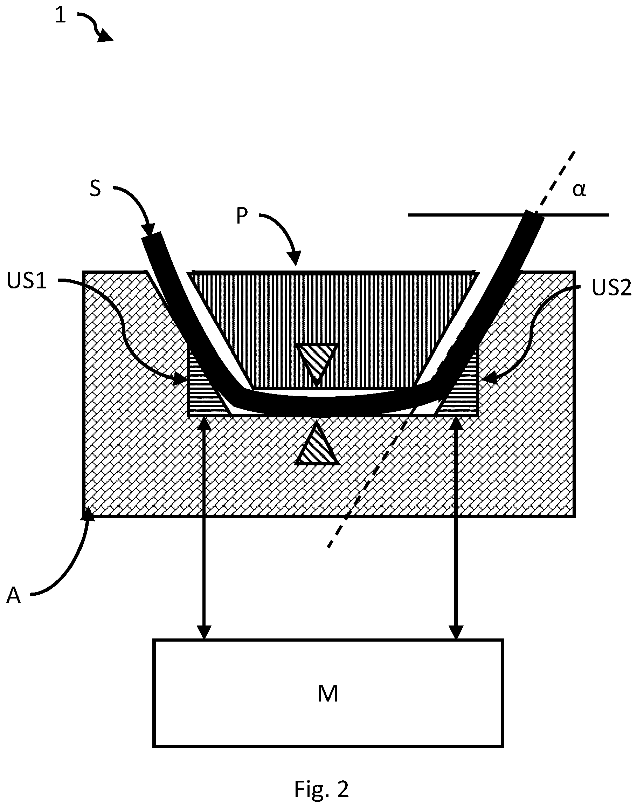

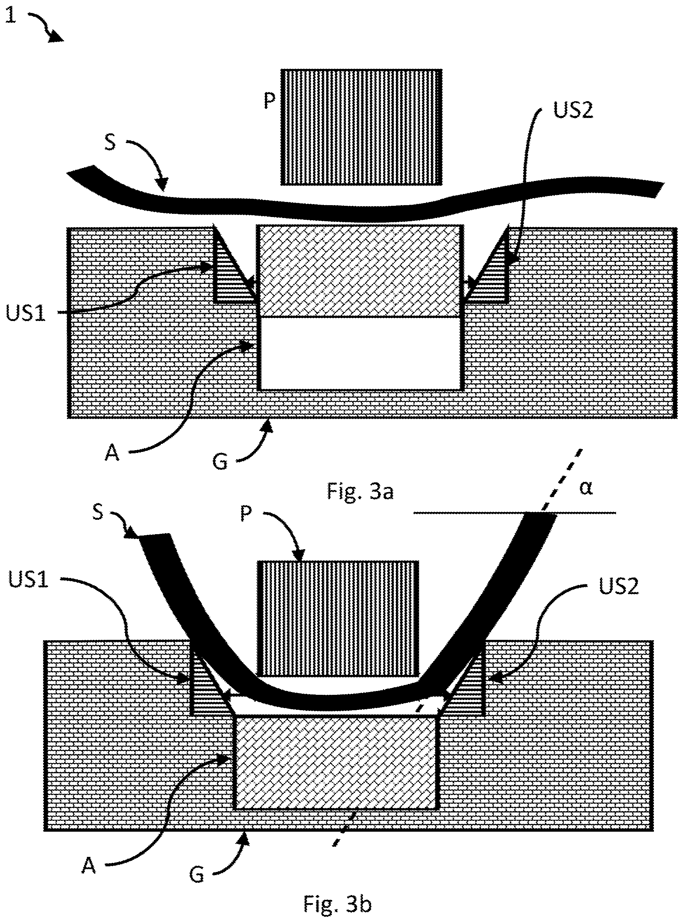

In einer Ausführungsform der Erfindung weist eine Vorrichtung 1 für ein medizinisches Behandlungsgerät zur Messung des Flusses von Flüssigkeiten in einem einzulegenden Lumen S eine Aufnahme D und einen ersten Ultraschallwandler US1 auf. Beispielhaft sei auf die Figur 1 ohne Lumen und die Figuren 2, 3a und 3b mit Lumen S verwiesen. Ultraschallwandler sind in verschiedenen Bauarten erhältlich, insbesondere sind jedoch von der Erfindung piezo-elektrische Wandler umfasst.

Das medizinische Behandlungsgerät ist dabei insbesondere ein Blutbehandlungsgerät, wie z.B. ein Gerät zur Oxygenierung von Blut, ein Gerät zur Entfernung von löslichen Bestandteilen aus Blut, wie z.B. ein Blutdialysegerät. Dialyse ist dabei weit zu verstehen und kann insbesondere Hämodialyse, Hämodiafiltration, Peritonealdialyse, Hämoultrafiltration, Apherese, etc., d.h. insbesondere jegliche Form der Nieren- oder Leberersatztherapie, bedeuten.

Die Aufnahme D in der Vorrichtung 1 weist eine erste Führungsseite A und eine zweite Führungsseite P auf, sodass ein eingelegtes Lumen S in seitlicher Projektion in etwa einen trapezförmigen Verlauf aufweist, wobei der erste Ultraschallwandler US1 an einer ersten Trapezecke angeordnet ist, und wobei der erste Ultraschallwandler US1 in Betrieb dazu ausgestaltet ist eine Messung in bzw. gegen Flussrichtung im Lumen S durchzuführen.

Es sei dabei angemerkt, dass die genaue Richtung der Messung mittels des ersten Ultraschallwandlers US1 der Ausgestaltung durch den Fachmann überlassen ist, sodass die in den Figuren skizzierte (dreieckig dargestellte) Anordnung nur beispielhaft ist. D.h. insbesondere kann der Ultraschallwandler auch so angeordnet sein, dass die Messrichtung nicht in Richtung eines der horizontal dargestellten Pfeile angeordnet ist, ebenso wäre es denkbar, dass in Richtung der gestrichelten Linie in Figur 2 gemessen werden könnte.

Weiterhin sei angemerkt, dass trapezförmig lediglich zur Beschreibung von räumlichen Orientierungen verwendet wird, um eine Führung des Lumens zu illustrieren, wobei Abschnitte aufeinandertreffen, die einen Winkel von ungleich 90° einschließen. In den Figuren ist dies durch den Winkel α verdeutlicht.

Zudem sei anmerkt, dass das Messprinzip unterschiedlich ausgestaltet sein kann. Neben dem Prinzip der Laufzeitmessung ist es auch möglich mit Doppler-Verfahren (Frequenzverschiebung durch (Rück-) Streuung am bewegten Objekt) Flussgeschwindigkeiten der zu untersuchenden (medizinischen) Flüssigkeit im Lumen S zu bestimmen, ohne jedoch hierauf beschränkt zu sein.

Diese Ausgestaltung stellt bei einfacher Handhabung eine hohe Messauflösung für verschiedene Lumendurchmesser bereit.

In einer Ausführungsform der Erfindung weist die Vorrichtung 1 weiterhin einen zweiten Ultraschallwandler US2 auf, wobei der zweite Ultraschallwandler US2 an einer zweiten Trapezecke angeordnet ist, und wobei der zweite Ultraschallwandler US2 in Betrieb dazu ausgestaltet ist eine Messung in bzw. gegen Flussrichtung im Lumen S in Gegenrichtung zum ersten Ultraschallwandler US1 durchzuführen.

Diese Ausgestaltung bietet den Vorteil, dass sowohl in Fluss- aus auch Gegenflussrichtung gemessen werden kann, sodass ein höhere Messgenauigkeit zur Verfügung gestellt werden kann. Eine solche Messung kann dabei entweder unabhängig, d.h. in Reflektion von jeweils einem Ultraschallwandler ins Lumen und von dort zurück zum selben Ultraschallwandler, oder abhängig, d.h. in Transmission von einem Ultraschallwandler durch das Lumen zum anderen Ultraschallwandler erfolgen. Dies kann abhängig vom Abstand der Ultraschallwandler zueinander, der Art des Lumens, der Messfrequenz, oder der zu messenden Flüssigkeit bzw. seiner Bestandteile etc. sein. Offensichtlich kann durch eine gezielte Steuerung auch ein wechselnder Betrieb von Ultraschallwandlern in Transmission bzw. Reflektion erfolgen.

Da nunmehr Messungen aus verschiedenen Richtungen möglich werden, können Messergebnisse sowohl in eine gemeinsame Berechnung einfließen, als auch zur Mittelung verwendet werden.

Gemäß einer Ausführungsform der Erfindung ist die zu vermessende Flüssigkeit Blut.

An die Messung von Eigenschaften von Blut werden besondere Anforderungen gestellt. Damit kann die Vorrichtung insbesondere für medizinische Blutbehandlungsgeräte verwendet werden, wie z.B. ein Gerät zur Oxygenierung von Blut, z.B. für Vorrichtungen zur Herz- und/oder Lungenunterstützung oder Ersatztherapie, ein Gerät zur Entfernung von (löslichen) Bestandteilen aus Blut, wie z.B. ein Blutdialysegerät. Dialyse ist dabei weit zu verstehen und kann insbesondere Hämodialyse, Hämodiafiltration, Peritonealdialyse, Hämoultrafiltration, Apherese, etc., d.h. insbesondere jegliche Form der Nieren- oder Leberersatztherapie, bedeuten.

Gemäß einer Ausführungsform der Erfindung ist die zu vermessende Flüssigkeit Blut, eine andere biologische Flüssigkeit oder Dialyse-Substituat oder eine medizinische Flüssigkeit wie ein Flüssigmedikament oder ein Gemisch der vorgenannten Flüssigkeiten.

Damit kann die Vorrichtung insbesondere für Blutbehandlungsgeräte, z.B. Dialyse- und Apheresegeräte, und im Kontext von extrakorporalen Blutkreislaufen, z.B. für Vorrichtungen zur Herz- und/oder Lungenunterstützung oder Ersatztherapie, verwendet werden.

Gemäß einer Ausführungsform der Erfindung ist die zu vermessende Flüssigkeit Dialysat oder eine andere physiologische Ersatzflüssigkeit oder eine medizinische Flüssigkeit wie ein Flüssigmedikament oder ein Gemisch der vorgenannten Flüssigkeiten.

Damit kann die Vorrichtung insbesondere für Blutbehandlungsgeräte wie Dialysegeräte, Dialysespendeanordnungen und Apheresegeräte, verwendet werden.

In einer weiteren Ausführungsform der Erfindung ist die zu vermessende Flüssigkeit Flüssignahrung z.B. zur Sondenernährung.

In einer weiteren Ausführungsform der Erfindung weist die Vorrichtung weiterhin eine Klemme auf, die ein eingelegtes Lumen S selektiv durch Abklemmen unterbrechen kann.

Damit wird ermöglicht, neben Messfunktionen auch Schaltfunktionen, z.B. in einem erkannten Fehlerfall, bereitzustellen. Beispielsweise kann Luft im Lumen S ein Grund sein, dass z.B. eine Behandlung mittels einer Blutbehandlungsvorrichtung gestoppt wird.

Weiterhin kann durch eine Bereitstellung einer Klemme in der Vorrichtung der Prozess des Einlegens eines Lumens vereinfacht werden, da häufig im weiteren Verlauf des Lumens S, z.B. bei einem Blutbehandlungsgerät, eine solche Klemme im Flussweg angeordnet sein muss. D.h., mit der integrierten Anordnung können Arbeitsschritte vermieden werden, wodurch die Rüstzeiten sinken.

Eine geeignete Klemme kann in die Vorrichtung 1 integriert sein. Beispielsweise kann eine Klemme - dargestellt als Dreiecke in Figur 2 - an geeigneter Stelle integriert sein. Bevorzugt wird durch die Klemme kein Druck auf die Ultraschallwandler US1, US2 ausgeübt.

Gemäß einer weiteren Ausführungsform der Erfindung ist das Trapez - wie in den Figuren durch den Winkel α gezeigt - gleichschenklig.

Mit einer gleichschenkligen Anordnung kann die Messtechnik spiegelbildlich und damit mit gleichartigen Teilen aufgebaut werden wodurch Entwicklungs- als auch Produktionskosten sinken.

In noch einer weiteren Ausführungsform der Erfindung weist die Vorrichtung weiterhin eine (externe) Auswerteeinrichtung M zur Steuerung und Auswertung von Ultraschallwandler(n) auf. Beispielsweise kann - wie in Figur 1 skizziert - die Auswerteeinrichtung M die Ultraschallwandler US1, US2 ansteuern und auch Daten / Messwerte von den Ultraschallwandlern US1, US2 erhalten.

D.h. mittels der Auswerteeinrichtung kann nun eine (integrierte) Baueinheit zur Verfügung gestellt werden.

Gemäß noch einer weiteren Ausführungsform der Erfindung weist die Vorrichtung weiterhin mindestens einen weiteren Sensor S1, S2, S3 zur Messung eines weiteren Parameters der Flüssigkeit auf. Dabei können die Sensoren S1, S2, einzeln oder auch als Gruppe S3 angeordnet sein. Beispielsweise kann der Sensor S1 ein Temperatursensor sein, während der Sensor S2 ein reflektiver optischer Sensor ist bzw. der Sensor S3 ein transmissiver optischer Sensor sein könnte. Mittels solcher Sensoren können z.B. Luftblasen, Bestandteile und deren Konzentrationen, Dichte, Temperatur, etc. bestimmt werden.

Durch die Integration weiterer Sensoren lassen sich kostengünstige Baugruppen mit weiteren Mess-Eigenschaften aufbauen.

In einer weiteren Ausführungsform der Erfindung ist die Vorrichtung weiterhin dazu eingerichtet Mikroblasen in der Flüssigkeit, welche sich in einem eingelegten Lumen S befindet, z.B. an Hand von Fluktuation in gemessenen Flüssen zu erkennen.

Mikroblasen können Zeichen beginnender Probleme als auch Fehler sein, sodass das Erkennen von Mikroblasen die Sicherheit erhöht. Hierbei kommt vorteilhaft zum Tragen, dass die Anordnung eine Messung an einem größeren Flüssigkeitsvolumen vorsieht als in den beinahe senkrechten (oder auch unter einem Winkel von 45°) bekannten Messanordnungen. Somit kann auch ein größeres Volumen zeitgleich auf die Gegenwart von Mikroblasen hin gemessen werden. In einer Variante der Ausführungsform wird die Gegenwart von Mikroblasen anhand von Fluktuationen im Messignal, aus welchem der Fluss ermittelt wird (z.B. Laufzeitdifferenz von Schallsignalen mit und entgegen der Flussrichtung), aber nicht zwingend direkt aus der somit gewonnen Messgröße allein ermittelt, sondern es wird eine zusätzliche Größe zur Auswertung der Fluktuationen im Messsignal herangezogen. Diese zusätzliche Messgröße kann z.B. eine Amplitudeninformation des übertragenen Ultraschallsignals sein.

Gemäß noch einer weiteren Ausführungsform der Erfindung sind - wie aus Figur 1 und 2 ersichtlich - die erste Führungsseite A und die zweite Führungsseite P relativ zueinander verschiebbar.

Hierdurch kann die Bandbreite von Lumendurchmessern besonders gut vergrößert werden. Ebenso kann der Anpressdruck geeignet einstellbar sein, sodass die Ultraschallwandler US1, US2 in einem günstigen Arbeitsbereich verwendet werden können.

In noch einer weiteren Ausführungsform der Erfindung übt die erste Führungsseite A und die zweite Führungsseite P auf ein eingelegtes Lumen S eine definierte Kraft, insbesondere eine einstellbare Kraft, aus.

Es sei weiter angemerkt, dass auch eine Verrastung vorgesehen sein kann, sodass z.B. ein bestimmter Anpressdruck gehalten werden kann.

Hierdurch kann der Ultraschallwandler in einem günstigen Arbeitsbereich Messungen mit hoher Genauigkeit zur Verfügung stellen.

Gemäß noch einer weiteren Ausführungsform der Erfindung ist eine Aufnahme für verschiedene Durchmesser von einlegbaren Lumen S ausgelegt.

Es sei angemerkt, dass die Bereitstellung einer Vorrichtung für Einweglumen deutliche Vorteile zur Verfügung stellt, da nunmehr nur noch wenige Teile einfach sterilisiert werden müssen.

In noch einer weiteren Ausführungsform der Erfindung - wie in Figur 3a und 3b dargestellt - kann die erste Führungsseite A und die zweite Führungsseite P schubladenartig gemeinsam relativ zu einem Gehäuse G verschoben werden. Durch diese schubladenförmige Ausgestaltung wird beim Einschieben der Schublade zusammen mit dem Gehäuse G die gewünschte Zwangsbahn für das Lumen S bereitgestellt, sodass auch in dieser Ausführungsform alle vorgenannten Vorteile realisiert werden können. Die Zwangsbahn ist zwar vorstehend als trapezförmig beschrieben, jedoch schließt dies nicht aus, dass auch eine W-Form oder eine V-Form oder eine S-Form in Abschnitten bereitgestellt wird.

Ohne Beschränkung der Allgemeinheit kann die Aufnahme D auch aus mehreren (Spritzguß-) Bauteilen aufgebaut sein. Beispielsweise kann ein wannenartiges Unterteil bereitgestellt werden, in das ein Oberteil, in das das Lumen S einfach (z.B. seitlich/normal zur Einlege-Richtung) eingelegt werden kann, (linear) eingeführt wird. Die Ultraschallwandler US1, US2 können dabei an dem Oberteil oder an dem Unterteil angeordnet sein. Wichtig ist nur, dass die Ultraschallwandler US1, US2 im zusammengeführten Zustand für eine Kopplung an dem Lumen S anliegen.

Ohne Beschränkung der Allgemeinheit kann eine solche Einführbewegung (oder auch ein Anpressdruck) manuell oder unterstützt oder automatisiert (mittels Federkräften, Elektroantrieben, Druckluft, etc.) bereitgestellt werden.

Gemäß einer weiteren Ausgestaltung der Erfindung wird auch ein Ensemble mit einer erfindungsgemäßen Vorrichtung zur Messung und einem Lumen zur Verfügung gestellt.

Diese Ausgestaltung stellt bei einfacher Handhabung eine hohe Messauflösung für verschiedene Lumendurchmesser bereit.

Gemäß einer Ausführungsform der Erfindung ist das Lumen S ein Einwegschlauch, insbesondere ein Einwegschlauch für eine medizinische Flüssigkeit, wie z.B. Blut, ein Dialysatzulauf, ein Permeatzulauf, ein Dialysatablauf, etc..

In einer Ausführungsform der Erfindung ist das Lumen S elastisch. Insbesondere kann das Lumen S aus einem geeigneten optisch transparenten Stoff hergestellt sein.

Damit lassen sich herkömmliche Schlauchsysteme als auch Einwegartikel auch mit der erfindungsgemäßen Vorrichtung kombinieren.

Es sei angemerkt, dass bei der Messung mit Ultraschall, d.h. mittels Ultraschallwandlern US1, US2 die Ankopplung ein wichtiger Aspekt ist. Die Ankopplung ist abhängig von der Impedanz zwischen Lumen S und Ultraschallwandlern US1, US2. Die Impedanz ist maßgeblich von der Anwesenheit von Luft abhängig. D.h. wird das Lumen S mit ausreichender Kraft an den / die Ultraschallwandler US1, US2 angedrückt, so ist die Anwesenheit von Luft zwischen dem Lumen S und den Ultraschallwandlern US1, US2 gering und es besteht eine gute Kopplung.

In bisherigen Klemmanordnungen, die eine Einkopplung oder Messung senkrecht zur Flussrichtung bzw. senkrechte, aber leicht versetzte (bis zu 45°) Anordnung vorsahen, bewirkte der Anpressdruck eine Verformung des Lumens, sodass dieser zwar besser an den Ultraschallwandlern anlag, jedoch war der Anpressdruck (hervorgerufen durch die Elastizität des Lumens) zugleich negativ für die Messung. Zudem war der Anpressdruck bei unterschiedlichen Lumen stark abhängig von der Größe des Lumens, der Materialstärke des Lumens als auch dem Material des Lumens. D.h., die Varianz der Lumen machte es bisher fast unmöglich für alle unterschiedlich verwendbaren Lumen ein ähnlich gutes Messergebnis bereitzustellen. Dies führte in der Vergangenheit dazu, dass nur eine geringe Untermenge für den Gebrauch mit einer bestimmten Messvorrichtung zugelassen war. Zusätzlich musste häufig eine Messanordnung auf den jeweiligen individuellen Schlauchtyp kalibriert werden, sodass auch bereits allein deshalb eine Schwierigkeit bestand.

Im Unterschied zum Stand der Technik wird nunmehr eine Orientierung der Messtrecke senkrecht zur Führung / Fixierung bereitgestellt, sodass zum einen die Problematik des Ankoppelns als auch des Messbereiches als auch der Variabilität der Lumen gelöst werden kann. Vielmehr ist es im Rahmen der erfindungsgemäßen Vorrichtung auch möglich einen Anpressdruck während einer laufenden Behandlung / Messung zu variieren. Je nach Messung kann z.B. ein Messsignal des Ultraschallwandlers oder eines Sensors S1...S3 rückgeführt werden, um z.B. den Anpressdruck variabel nachzuführen, sodass ein gewünschter Signal Hub realisiert werden kann.

Da in der Erfindung nunmehr eine Messung in / gegen Fluss-Richtung ermöglicht wird, können die Messintervalle für Laufzeitdifferenzen vergrößert werden. Somit ist ein besseres Signal zu Rauschverhältnis erreichbar. Durch die längere Ausgestaltung der Messstrecke sinken zudem die Anforderungen an die nötige Zeitauflösung bei der Messung der Laufzeitdifferenz, welche günstigere Realisierungen ermöglicht.

Anders als bei bisherigen Ansätzen ist es zudem möglich Mikroblasen nicht nur qualitativ zu erkennen, sondern auch zu quantifizieren. Durch die längere Messstrecke und Effekte die beim Ein- und Austreten der Luft, sowie während der längeren Verweildauer in der Messstrecke lässt sich Luft besser detektieren und quantifizieren. Durch ein integrales Volumen welches in der Messstrecke betrachtet - im Gegensatz zu einem im bisherigen Stand der Technik genutzten lediglich 2-dimensionalen Querschnitte - ergibt sich eine einfachere Bestimmung von Luftmengen.

Das Verhältnis von Schlauchwandung zu Messtrecke wird bei der Anpassung optimiert. Dies hat zur Folge, dass die Messungen unabhängiger von Toleranzen der Schlauchwandungen sind. Insbesondere ist ein größerer Anteil der Messstrecke nicht verformt, da an dem größten keine Ankopplung der Sensorik benötigt wird.

## Patentansprüche

1. Vorrichtung (1) für ein medizinisches Behandlungsgerät zur Messung des Flusses von Flüssigkeiten in einem einzulegenden Lumen (S), aufweisend eine Aufnahme (D) und einen ersten Ultraschallwandler (US1), wobei die Aufnahme (D) in der Vorrichtung (1) eine erste Führungsseite (A) und eine zweite Führungsseite (P) aufweist, sodass ein eingelegtes Lumen (S) in seitlicher Projektion in etwa einen trapezförmigen Verlauf aufweist, wobei der erste Ultraschallwandler (US1) an einer ersten Trapezecke angeordnet ist, und wobei der erste Ultraschallwandler (US1) im Betrieb dazu ausgestaltet ist, eine Messung in bzw. gegen Flussrichtung im Lumen (S) durchzuführen.

2. Vorrichtung (1) zur Messung nach Anspruch 1, **dadurch gekennzeichnet, dass** die Vorrichtung (1) weiterhin einen zweiten Ultraschallwandler (US2) aufweist, wobei der zweite Ultraschallwandler (US2) an einer zweiten Trapezecke angeordnet ist, und wobei der zweite Ultraschallwandler (US2) im Betrieb dazu ausgestaltet ist, eine Messung in bzw. gegen Flussrichtung im Lumen (S) in Gegenrichtung zum ersten Ultraschallwandler (US1) durchzuführen.

3. Vorrichtung zur Messung nach einem der vorhergehen Ansprüche, **dadurch gekennzeichnet, dass** die Flüssigkeit Blut, eine andere biologische Flüssigkeit oder eine physiologische Ersatzflüssigkeit wie Dialysat, Dialyse-Substituat oder eine medizinische Flüssigkeit wie ein Flüssigmedikament oder ein Gemisch der vorgenannten Flüssigkeiten oder eine Flüssignahrung ist.

4. Vorrichtung zur Messung nach einem der vorhergehen Ansprüche, **dadurch gekennzeichnet, dass** die Vorrichtung weiterhin eine Klemme aufweist, die ein eingelegtes Lumen selektiv durch Abklemmen unterbrechen kann.

5. Vorrichtung zur Messung nach einem der vorhergehen Ansprüche, **dadurch gekennzeichnet, dass** das Trapez gleichschenklig ist.

6. Vorrichtung zur Messung nach einem der vorhergehen Ansprüche, **dadurch gekennzeichnet, dass** die Vorrichtung weiterhin eine externe Auswerteeinrichtung (M) zur Steuerung und Auswertung von Ultraschallwandlern aufweist.

7. Vorrichtung zur Messung nach einem der vorhergehen Ansprüche, **dadurch gekennzeichnet, dass** die Vorrichtung weiterhin mindestens einen weiteren Sensor (S1, S2, S3) zur Messung eines weiteren Parameters der Flüssigkeit aufweist.

8. Vorrichtung zur Messung nach einem der vorhergehen Ansprüche, **dadurch gekennzeichnet, dass** die Vorrichtung weiterhin dazu eingerichtet ist, Mikroblasen in der medizinischen Flüssigkeit, welche sich in einem eingelegten Lumen befindet, zu erkennen.

9. Vorrichtung zur Messung nach Anspruch 8, **dadurch gekennzeichnet, dass** die Vorrichtung weiterhin dazu eingerichtet ist, Mikroblasen in der medizinischen Flüssigkeit an Hand von Fluktuation in gemessenen Flüssen zu erkennen.

10. Vorrichtung zur Messung nach einem der vorhergehen Ansprüche, **dadurch gekennzeichnet, dass** die erste Führungsseite (A) und die zweite Führungsseite (P) relativ zueinander verschiebbar sind.

11. Vorrichtung zur Messung nach Anspruch 10, **dadurch gekennzeichnet, dass** die erste Führungsseite (A) und die zweite Führungsseite (P) auf ein eingelegtes Lumen (S) eine definierte Kraft ausüben.

12. Vorrichtung zur Messung nach Anspruch 10 oder 11, **dadurch gekennzeichnet, dass** die erste Führungsseite (A) und die zweite Führungsseite (P) auf ein eingelegtes Lumen (S) eine einstellbare Kraft ausüben.

13. Vorrichtung zur Messung nach einem der vorhergehen Ansprüche, **dadurch gekennzeichnet, dass** die eine Aufnahme (D) für verschiedene Durchmesser von einlegbaren Lumen (S) ausgelegt ist.

14. Vorrichtung zur Messung nach einem der vorhergehenden Ansprüche, **dadurch gekennzeichnet, dass** die erste Führungsseite (A) und die zweite Führungsseite (P) schubladenartig gemeinsam relativ zu einem Gehäuse (G) verschoben werden können.

15. Ensemble mit einer Vorrichtung zur Messung nach einem der vorhergehen Ansprüche und einem Lumen (S).

16. Ensemble nach Anspruch 15, **dadurch gekennzeichnet, dass** das Lumen (S) ein Einwegschlauch ist.

17. Ensemble nach Anspruch 15 oder 16, **dadurch gekennzeichnet, dass** das Lumen (S) elastisch ist.

## Claims

1. A device (1) for a medical treatment apparatus for measuring the flow of liquids in a lumen (S) to be inserted, having a receptacle (D) and a first ultrasonic transducer (US1), wherein the receptacle (D) in the device (1) has a first guide side (A) and a second guide side (P), so that an inserted lumen (S) has approximately a trapezoidal course in lateral projection, wherein the first ultrasonic transducer (US1) is arranged at a first trapezoid corner, and wherein the first ultrasonic transducer (US1) in operation is designed to perform a measurement in or against the direction of flow in the lumen (S), respectively.

2. The device (1) for measuring according to claim 1, **characterized in that** the device (1) furthermore has a second ultrasonic transducer (US2), wherein the second ultrasonic transducer (US2) is arranged at a second trapezoid corner, and wherein the second ultrasonic transducer (US2) in operation is designed to perform a measurement in or against the direction of flow in the lumen (S), respectively, in the opposite direction to the first ultrasonic transducer (US1).

3. The device for measuring according to one of the preceding claims, **characterized in that** the liquid is blood, another biological liquid, or a physiological replacement liquid, such as dialysate, dialysis substituate, or a medical liquid, such as a liquid medicine or a mixture of the above-mentioned liquids or liquid food.

4. The device for measuring according to one of the preceding claims, **characterized in that** the device furthermore has a clamp, which can selectively interrupt an inserted lumen by means of cross-clamping.

5. The device for measuring according to one of the preceding claims, **characterized in that** the trapezoid is isosceles.

6. The device for measuring according to one of the preceding claims, **characterized in that** the device furthermore has an external evaluation means (M) for controlling and evaluating ultrasonic transducers.

7. The device for measuring according to one of the preceding claims, **characterized in that** the device furthermore has at least one further sensor (S1, S2, S3) for measuring a further parameter of the liquid.

8. The device for measuring according to one of the preceding claims, **characterized in that** the device is furthermore configured to recognize micro-bubbles in the medical liquid, which is located in an inserted lumen.

9. The device for measuring according to claim 8, **characterized in that** the device is furthermore configured to recognize micro-bubbles in the medical liquid on the basis of fluctuation in measured flows.

10. The device for measuring according to one of the preceding claims, **characterized in that** the first guide side (A) and the second guide side (P) can be displaced relative to one another.

11. The device for measuring according to claim 10, **characterized in that** the first guide side (A) and the second guide side (P) exert a defined force on an inserted lumen (S).

12. The device for measuring according to claim 10 or 11, **characterized in that** the first guide side (A) and the second guide side (P) exert a settable force on an inserted lumen (S).

13. The device for measuring according to one of the preceding claims, **characterized in that** the one receptacle (D) is designed for various diameters of insertable lumens (S).

14. The device for measuring according to one of the preceding claims, **characterized in that** the first guide side (A) and the second guide side (P) can be displaced jointly relative to a housing (G) in a drawer-like manner.

15. An ensemble with a device for measuring according to one of the preceding claims and a lumen (S).

16. The ensemble according to claim 15, **characterized in that** the lumen (S) is a disposable hose.

17. The ensemble according to claim 15 or 16, **characterized in that** the lumen (S) is elastic.

## Revendications

1. Dispositif (1) pour un appareil de traitement médical pour mesurer le débit de liquides dans une lumière à mettre en place (S), comportant un logement (D) et un premier transducteur d'ultrasons (US1), sachant que le logement (D) dans le dispositif (1) comporte un premier côté de guidage (A) et un deuxième côté de guidage (P), de telle qu'une lumière mise en place (S) comporte en projection latérale un profil à peu près trapézoïdal, sachant que le premier transducteur d'ultrasons (US1) est disposé sur un premier angle de trapèze et sachant que le premier transducteur d'ultrasons (US1) est conçu en fonctionnement pour exécuter une mesure dans ou contre le sens d'écoulement dans la lumière (S).

2. Dispositif de mesure (1) selon la revendication 1, **caractérisé en ce que** le dispositif (1) comporte en plus un deuxième transducteur d'ultrasons (US2), sachant que le deuxième transducteur d'ultrasons (US2) est disposé sur un deuxième angle de trapèze, et sachant que le deuxième transducteur d'ultrasons (US2) est conçu en fonctionnement pour exécuter une mesure dans ou contre le sens d'écoulement dans la lumière (S) dans le sens contraire au premier transducteur d'ultrasons (US1).

3. Dispositif de mesure selon l'une quelconque des revendications précédentes, **caractérisé en ce que** le liquide est du sang, un autre liquide biologique ou un liquide de substitution physiologique comme du dialysat, un substitut de dialyse ou un liquide médical comme un médicament liquide ou un mélange des liquides précités ou un aliment liquide.

4. Dispositif de mesure selon l'une quelconque des revendications précédentes, **caractérisé en ce que** le dispositif comporte en plus une pince qui peut interrompre sélectivement par desserrage une lumière mise en place.

5. Dispositif de mesure selon l'une quelconque des revendications précédentes, **caractérisé en ce que** le trapèze est isocèle.

6. Dispositif de mesure selon l'une quelconque des revendications précédentes, **caractérisé en ce que** le dispositif comporte en plus un système d'évaluation externe (M) pour commander et évaluer des transducteurs d'ultrasons.

7. Dispositif de mesure selon l'une quelconque des revendications précédentes, **caractérisé en ce que** le dispositif comporte en plus au moins un autre capteur (S1, S2, S3) pour la mesure d'un autre paramètre du liquide.

8. Dispositif de mesure selon l'une quelconque des revendications précédentes, **caractérisé en ce que** le dispositif est en plus agencé pour identifier des microbulles dans le liquide médical, lequel se trouve dans une lumière mise en place.

9. Dispositif de mesure selon la revendication 8, **caractérisé en ce que** le dispositif est en plus agencé pour identifier des microbulles dans le liquide médical à l'aide de la fluctuation dans les débits mesurés.

10. Dispositif selon l'une quelconque des revendications précédentes, **caractérisé en ce que** le premier côté de guidage (A) et le deuxième côté de guidage (P) peuvent être déplacés l'un par rapport à l'autre.

11. Dispositif de mesure selon la revendication 10, **caractérisé en ce que** le premier côté de guidage (A) et le deuxième côté de guidage (P) exercent une force définie sur la lumière mise en place (S).

12. Dispositif de mesure selon la revendication 10 ou 11, **caractérisé en ce que** le premier côté de guidage (A) et le deuxième côté de guidage (P) exercent une force réglable sur la lumière mise en place (S).

13. Dispositif de mesure selon l'une quelconque des revendications précédentes, **caractérisé en ce qu'**un logement (D) est conçu pour des diamètres différents de lumière pouvant être mise en place (S).

14. Dispositif de mesure selon l'une quelconque des revendications précédentes, **caractérisé en ce que** le premier côté de guidage (A) et le deuxième côté de guidage (P) peuvent être déplacés ensemble à la manière d'un tiroir par rapport à un boîtier (G).

15. Ensemble avec un dispositif de mesure selon l'une quelconque des revendications précédentes et une lumière (S).

16. Ensemble selon la revendication 15, **caractérisé en ce que** la lumière (S) est un tuyau à usage unique.

17. Ensemble selon la revendication 15 ou 16, **caractérisé en ce que** la lumière (S) est élastique.
